# EUROPEAN PATENT APPLICATION

(11) **EP 1 484 304 A1**
(43) Date of publication of application: **08.12.2004**
(21) Application number: 03011190.0
(22) Date of filing: 28.05.2003
(51) Int. Cl.: C07C 51/353, C07C 67/343

(54) **Process for the preparation of fluorophenylalkylene acid derivatives**

(71) Applicant: DAINIPPON INK AND CHEMICALS, INC., Itabashi-ku Tokyo (JP)
(72) Inventor: Döhler, Thomas, Dr. c/o DIC Berlin, 13403 Berlin (DE); Pitharth, Cornelia, Dr. c/o DIC Berlin, 13403 Berlin (DE); Grahe, Gerwald, Dr. c/o DIC Berlin, 13403 Berlin (DE); Frings, Rainer Bruno, Dr. c/o DIC Berlin, 13403 Berlin (DE)
(74) Representative: Albrecht, Thomas, Dr.

(57) **Abstract**

The invention relates to the preparation of fluorophenylalkylene acid derivatives of the formulae (4-1)-(4-3): 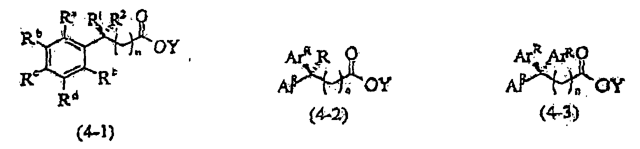 by reaction between fluoroarylmetal halides of formulae (1-1) or (1-2) with carboxylic acid derivatives of formulae (2-1)-(2-3): 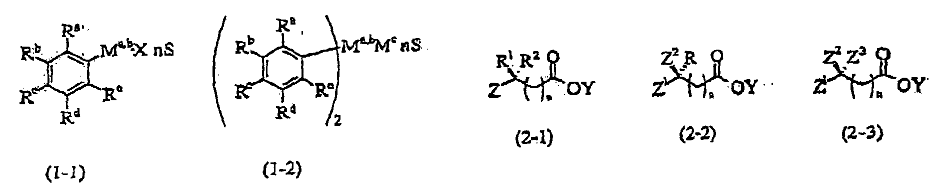 in the presence of at least one transition metal catalyst of formulae (3-1)-(3-7): 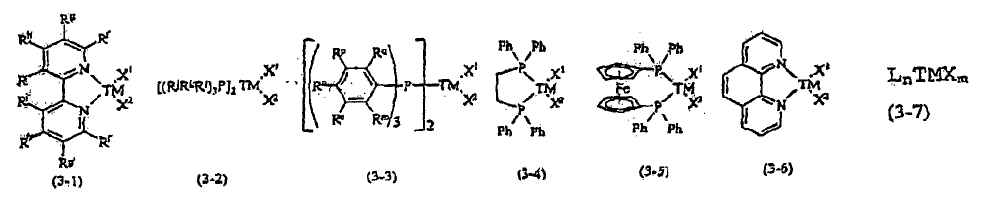 in the above formulae, the variables have the meanings given in the description.

## Description

Phenylacetic acid and -acid esters and their derivatives, particularly, those with one or more than one substituent at the phenyl ring are interesting starting compounds for active components in drugs and pesticides. Well-documented is the use of phenylacetic acids e.g. for the preparation of dibenzocycloheptatriene-type drugs, as described in DE 1593314 and for analgetica of the "fenac"- and "fenbufen"-type.
There are various processes for the preparation of unsubstitued and aryl substituted phenylacetic acid and its derivatives known. A. Burger and S. Avakian described the preparation of (4-methoxyphenyl)acetic acid from 4-methoxybenzoic acid via the corresponding α-diazoketone and its Wolff-rearrangement into the phenylacetic acid. A. McKillop et al. described the oxidative rearrangement of many aryl-substituted acetophenones, mediated by Tl(NO₃)₃ on Montmorillonit-K10 into the corresponding phenylacetic acid esters in J. Am. Chem. Soc. 95, 3340(1973). Other well-known methods are the hydrolysis of benzylnitriles, the reaction of benzylmagnesium halides with CO₂ and the Willgerodt-Kindler reaction, in which acetophenones are converted first with elementary sulfur in presence of a secondary amine, preferably morpholine, into a thioamide, which is then hydrolysed into the corresponding phenylacetic acid. All these processes have severe draw-backs under economical and ecological aspects, as toxic reagents are used, multi-step reactions are necessary, considerable amounts of toxic non-biogradable waste are produced and most reactions are dependent on the electronic nature and the position of the aryl-substituents with respect to the formation and the yield of the desired phenylacetic acid or its primary derivative.

As there is a pressing need for technologically, economically and ecologically more favourable methods for the preparation of phenylacetic acids, some patents have claimed approaches other than the above-mentioned. GB 820083 describes the preparation of substituted carboxylic acids, esters or salts, by reaction of Grignard-compounds with an α-halocarboxylic derivative like acid, ester or salt. The reaction is carried out under reflux without any catalyst. As the starting material contains carbonyl groups side reactions are possible. Organozinc compounds tolerate a number of functional groups, especially carbonyl functions, that should not be involved in the coupling process (Diederich, Stang, *Metal-catalyzed Cross-coupling Reactions,* Wiley-VCH **1998,** p. 18). This fact was picked up by *Klingstedt* and *Frejd (Nickel-catalyzed Synthesis of Arylacetic Esters from Arylzinc Chlorides and Ethyl Bro*moacetate, Klingstedt, T.; Frejd, T.; Organometallics **1983,** *2*, p. 598). The first method (reaction of aryl halide and lithiated ester) described in this paper is not practical for a larger scale, because a stoichiometric amount of nickel is necessary.
The therein described second method deals with a Reformatsky-type coupling, where an aryl halide reacts with bromo-2-(ethylacetato)zink(II), the Reformatsky reagent of ethyl bromoacetate. This process can be carried out in the presence of a catalytical amount of nickel(0). Therefore exist the following requirements: the use of HMPA, which is very toxic, and the preformation of the nickel(0) catalyst, because the Reformatsky-reagent is unable to reduce nickel(II) (precursor of the catalytic active species) to nickel(0). The latter is realized by additional reducing reagents or simply the use of mostly very air-sensitive nickel(0) complexes.
The third method bases on the coupling of arylzinc halides with ethyl bromoacetate in the presence of a nickel(II) catalyst. This catalyst is generated in situ by addition of one equiv. bis(acetylacetonato)nickel(II) and one equiv. phosphine ligand (PPh₃ or CyPh₂P) . Nevertheless, there are problems with relative low yields and side products, like biaryls.
US 2002/0062041 A1 describes the preparation of arylacetic esters by carbonylation of substituted benzyl chlorides in the presence of an primary alcohol R-OH, whereas R is the introduced group of the ester function. This process is carried out at 20-30bar CO and 130-150°C in an dipolar aprotic solvent (like N-methylpyrrolidone) and requires a transition metal catalyst, which contains a Group 8 metal (preferably ruthenium).

EP-A-1 191 008 and EP-A-1 201 632 describe the use of phenylacetic acids as starting products for the preparation of new liquid crystals, particularly those with one or more fluorosubstituents on the aryl-ring. Starting from the prior art described above, it is the object of the invention to provide a straight-forward one-pot preparation for these acids, respectively their esters, which makes use only of easily available educts, particularly various halo-fluoroaryls and both α- and β-haloacetic esters, which are coupled together by a Ni-catalyzed Zn-mediated reaction in high yield and purity. Thus, the invention aims at achieving a simple reaction sequence, a short reaction-time, good yields and selectivities, an easy product isolation with lower amounts of waste, use of commercial starting products and a wide versatility with respect to the aryl-substituents.

This invention relates to a process for the preparation of fluorophenylalkylencarboxylic acid derivatives (4-1) to (4-3): 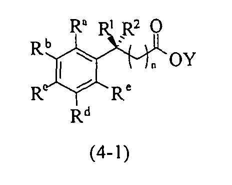 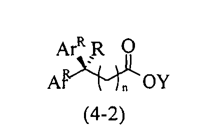 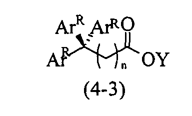 wherein R^{a} to R^{e} are H, F, alkyl, alkoxy, fluoroalkyl, cycloalkyl, fluorocycloalkyl, aryl, fluoroaryl, alkenyl, alkynyl, R, R¹ and R² are hydrogen atoms or alkyl, cycloalkyl, aryl, alkenyl and alkynyl, which can be substituted by alkyl and alkoxy groups,
n is from 0 to 10,
Y is alkyl, aryl, Li, Na, K or a group II metal (except Ra),
Ar^{R} is an aryl group substituted by H, F, alkyl, fluoroalkyl, cycloalkyl, fluorocycloalkyl, aryl, fluoroaryl, alkenyl, alkynyl;
wherein fluoroarylmetal halides (Ar^{R}M^{a,b}X)_{y}·nS (1-1) resp. (Ar^{R}M^{a,b}M^{c})_{y}·nS (1-2) : 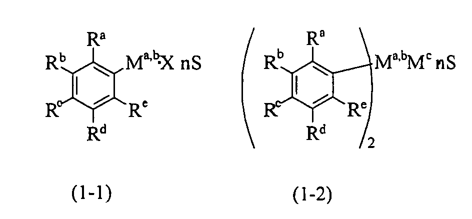 wherein R^{a}, R^{b}, R^{c}, R^{d} and R^{e} are as defined above, wherein in (1-1) M^{a} and M^{b} are Li, Mg, Zn, Cu(II) and in (1-2) M^{a} and M^{b} are Li, Mg or Zn, X is a Cl, Br or I, S is an optionally substituted dialkyl or alicyclic ether
are reacted with carboxylic acid derivatives RₚZ₍₃₋ₚ₎(CH₂)ₙCOOY (2-1)~(2-3) 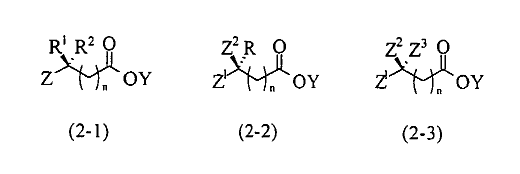 wherein Z, Z¹, Z² and Z³ are Cl, Br or I, n and Y are as defined above, p is 0 to 3,
in the presence of at least a transition metal catalyst LₙTMX¹X² selected from the group (3-1)~(3-6)

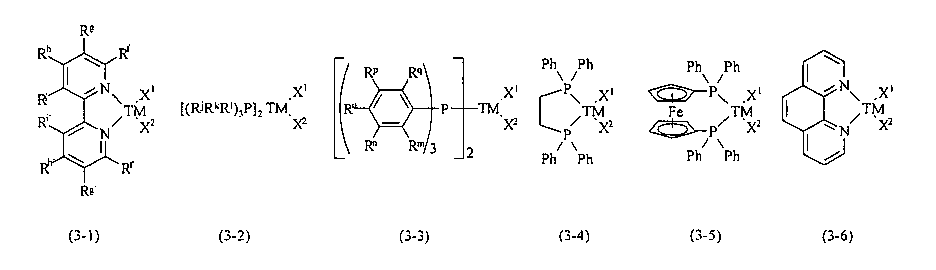

LₙTMXₘ (3-7)

wherein TM is divalent or zerovalent nickel, palladium or platinum,
R^{f}, R^{g}, R^{h}, Rⁱ, R^{f'}, R^{g'}, R^{h'} and R^{i'} are hydrogen, alkyl, alkoxy, perfluoroalkyl, perfluoroalkoxy, alkenyl, alkynyl or halogen (except At),
R^{j}, R^{k} and R^{l} are alkyl, alkoxy, perfluoroalkyl, perfluoroalkoxy, cycloalkyl, cycloalkoxy, perfluorocycloalkyl and perfluorocycloalkoxy.
R^{m}, Rⁿ, R^{o}, R^{p} and R^{q} are hydrogen, alkyl, alkoxy, perfluoroalkyl, perfluoroalkoxy, alkenyl, alkynyl or halogen (except At), and wherein, when TM is divalent, X¹ and X² are inorganic ionic ligands,
and when TM is zerovalent, X¹ and X² are inorganic or organic donorligands,
and wherein in (3-7) m and n are integers from 1 to 6, the ligand L is alkyl phosphine, aryl phosphine, alkyl aryl phosphine, bipyridyl or phenanthroline, substituted by hydrogen, alkyl, alkoxy, perfluoroalkyl, perfluoroalkoxy, cycloalkyl, cycloalkoxy, perfluorocycloalkyl, perfluoroalkoxy and perfluorocycloalkyl, alkenyl, alkynyl, chlorine, bromine or iodine;
the ligands can be bridged by substituted or unsubstituted alkyl groups, aryl groups and ferrocene;
in case TM is a divalent metal, X is an inorganic ionic ligand like chloride, bromide, iodide, hydride, sulfate, sulfite, nitrate, amide or amide substituted by alkyl, cycloalkyl, aryl or organic carboxylate;
X is alkyl, cycloalkyl, aryl or part of an ionic chelate ligand;
in case TM is a zerovalent metal, X can be an inorganic or organic donor ligand.

As mentioned above, the fluoroarylmetal compound is described by the general formulae (1) 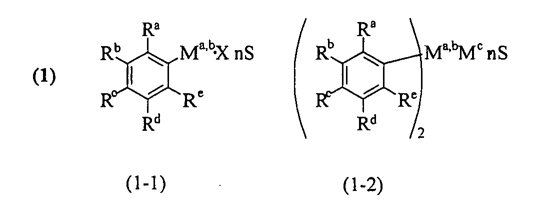 wherein in (1-1) M^{a} and M^{b} are Li, Mg, Zn, Cu(II). In the case of Li, the metal is not X-substituted. In (1-2) M^{a} and M^{b} are Li, Mg or Zn, M^{c} is Cu(I). X is a chlorine, bromine or iodine. R^{a} to R^{e} are H, F, alkyl, fluoroalkyl, cycloalkyl, fluorocycloalkyl, aryl, fluoroaryl, alkenyl, alkynyl. S is a dialkyl or alicyclic ether. The cyclic ether consists of a five or six membered ring which is composed of carbon atoms except one, which is oxygen. In the case of the six membered ring the carbon atom in position 4 can be replaced by nitrogen, which can be substituted by H, alkyl, fluoroalkyl, cycloalkyl, fluorocycloalkyl, aryl, fluoroaryl, alkenyl, alkynyl. The carbon ring atoms can be substituted by alkyl, alkoxy, aryl, aryloxy groups.

The term "alkyl" as used herein relates to an alkyl group having 1 to 25 carbon atoms.

The term "alkenyl" as used herein describes an unsaturated hydrocarbon group having 2 to 25 carbon atoms, wherein the position of the double bond(s) is not subject to any limitation.

The term "alkynyl" as used herein relates to hydrocarbons with one or more triple bonds having 2 to 25 carbon atoms, wherein the position of the triple bond(s) is not subject to any limitation.

The term "cycloalkyl" as used herein relates to cyclic alkyl groups having 3 to 8 carbon atoms.

The term "aryl" as used herein relates to aromatic groups such as phenyl and naphthyl groups.

The reaction is carried out in S as the reaction medium in the temperature range of -80°C to 260°C, depending on the melting and boiling point of S. Beside S a saturated cyclic, aromatic or non-cyclic hydrocarbon or mixtures thereof can be used as reaction medium.
The organometallic aryl metal compound is generated by reaction of the corresponding aryl halide ArX and M^{a} (M^{a}=Mg, Li; **(2),** equ. I). In the case of Cu and Zn this compound is obtained by transmetalation of ArM^{a}X using metal halide M^{b}X (M^{b}=Cu(II), Zn; **(2),** equ. II). This procedure is also practicable for M^{b}=Mg, when M^{a} is Li.

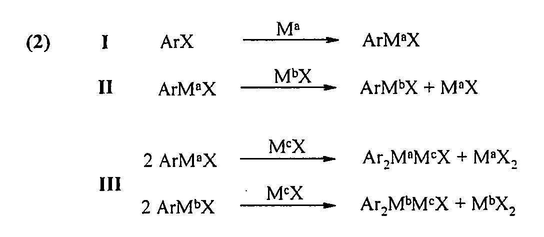

The described method is also valid for the syntheses of diaryl metalates, when M^{a} and M^{b} are Li respectively Mg, and M^{c} is Cu(I) (**(2)**, equ. III). It will be evident that when the metal has a differing valence, the formula must be modified to correspond. The in situ produced aryl metal halides must be handled between -80°C and reflux temperature of the solvent, depending on the stability of the organometallic species. Normal working temperature is between -80°C and 200°C, preferably between 20°C and 70°C.

The carboxylic coupling component is described by the general formulae (3) 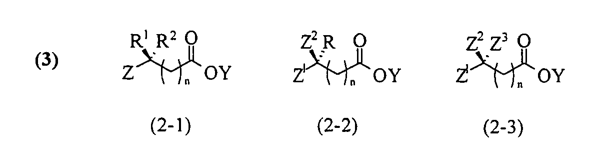 wherein Z, Z¹, Z² and Z³ are halogen atoms (which can be different from each other), except fluorine and astatine. R, R¹ and R² are preferably hydrogen atoms, but can also be alkyl, cycloalkyl, aryl, alkenyl and alkynyl, which can be substituted by alkyl and alkoxy groups. Y is alkyl, aryl, alkali metal (except Rb, Cs and Fr) or a group II metal (except Ra). n is an integer from 0 to 10, preferably 0 and 1.

As the formation of the required products via thermal metal halide elimination is unselective and accompanied by formation of side products, the coupling process was transformed to transition metal-catalyzed cross coupling. The choice of appropriate conditions minimizes the formation of side products. The advantage of the herein described process is the very high conversion and selectivity to the required arylalkylencarboxylic acid derivative. The formation of biphenyls, which are always formed by application of established methods, is completely avoided. Furthermore, this method is adaptable to a wide range of substrates. The used catalysts are easy to prepare and very cheap. As the used catalysts have high activity, this process can be carried out with high performance at room temperature, that means without additional energy effort for refluxing the reaction mixture. This catalyst system is described by the general formulas (3-1) to (3-7):

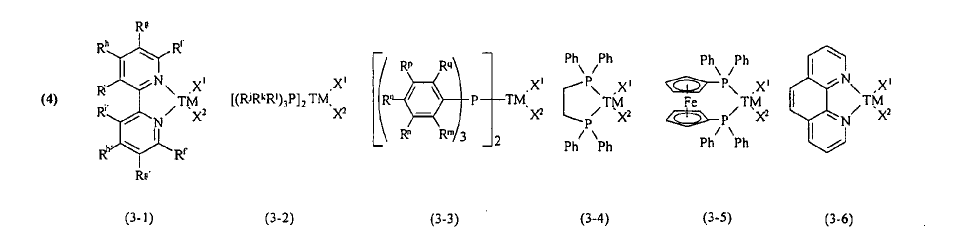

LₙTMXₘ (3-7)

wherein TM ("transition metal") is divalent or zerovalent nickel, palladium or platinum. In the case of divalent TM, X¹ and X² are inorganic ionic ligands like halide, hydride, sulfate, sulfite, nitrate, nitrite, substituted amide (H, alkyl, cycloalkyl, aryl) or organic carboxylates like acetates and propionates. Furthermore X¹ and X² can be: alkyl, cycloalkyl, aryl. The donor atoms X¹ and X² can be a part of an ionic chelate ligand like diketonate, e.g. penta-2,4-dionate, which can be substituted by alkyl, cycloalkyl, aryl and fluorine. It is evident, that a combination of both is possible.
In the case of zerovalent TM, X¹ and X² are inorganic or organic donorligands like carbon monoxide, amines (substituted by H, alkyl, cycloalkyl, aryl), phosphines (substituted by H, alkyl, cycloalkyl, perfluoroalkyl, aryl, perfluoroaryl, aryl sulfonate), ethers (non-cyclic, alicyclic, aromatic), monodentate olefines, bidentate olefines (butadiene, cyclooctadiene) or polydentate olefines (aromatic/heteroaromatic compounds, cyclooctatetraene). R^{f}, R^{g}, R^{h}, Rⁱ, R^{f'}, R^{g'}, R^{h'} and R^{i'} are hydrogen, alkyl, alkoxy, perfluoroalkyl, perfluoroalkoxy, alkenyl, alkynyl or halogen (except At). R^{j}, R^{k} and R^{l} are alkyl, alkoxy, perfluoroalkyl, perfluoroalkoxy, cycloalkyl, cycloalkoxy, perfluorocycloalkyl and perfluorocycloalkoxy. R^{m}, Rⁿ, R^{o}, R^{p} and R^{q} are hydrogen, alkyl, alkoxy, perfluoroalkyl, perfluoroalkoxy, alkenyl, alkynyl or halogen (except At).

In formula (3-7) shown above, TM is a transition metal and has the same meaning as in formulae (3-1) to (3-6). Further, m and n are integers from 1 to 6, the ligand L is alkyl phosphine, aryl phosphine, alkyl aryl phosphine, bipyridyl or phenanthroline, substituted by hydrogen, alkyl, alkoxy, perfluoroalkyl, perfluoroalkoxy, cycloalkyl, cycloalkoxy, perfluorocycloalkyl, perfluoroalkoxy and perfluorocycloalkyl, alkenyl, alkynyl, chlorine, bromine or iodine;
the ligands can be bridged by substituted or unsubstituted alkyl groups, aryl groups and ferrocene;
in case TM is a divalent metal, X is an inorganic ionic ligand like chloride, bromide, iodide, hydride, sulfate, sulfite, nitrate, amide or amide substituted by alkyl, cycloalkyl, aryl or organic carboxylate;
X is alkyl, cycloalkyl, aryl or part of an ionic chelate ligand;
in case TM is a zerovalent metal, X can be an inorganic or organic donor ligand.

In the case of the preparation of fluoro-substituted phenylacetic esters the phosphine-free complex bis(acetylacetonato)-2,2'-bipyridinenickel(II) represents an high efficient and selective catalyst, which avoids the formation of biaryls. The used combination of the starting materials, the catalyst and the final product is new. The surprising effect from that combination is, the reaction is mild and easy treated.

The herein described catalytic coupling process can be described by the general equations (5): 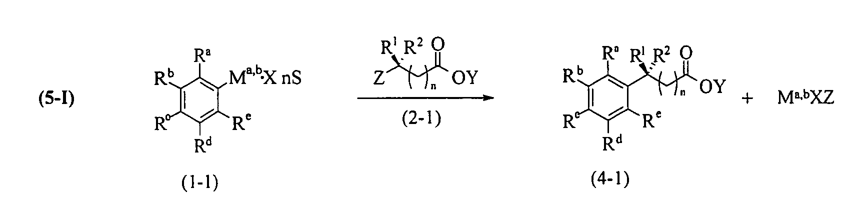 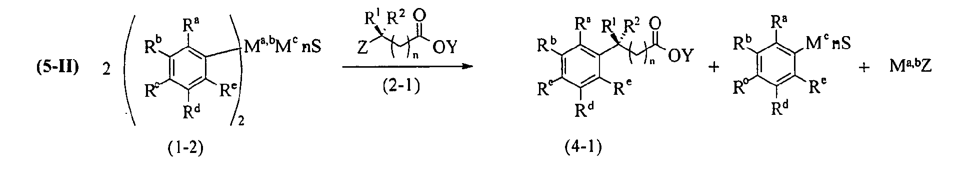 wherein all substituents/groups are as defined above.

The use of dihalo and trihalo esters enables the introduction of two, respectively three aromatic substituents to the ester group. The substituent Ar^{R} relates the above described aromatic group, which is substituted by R^{a}, R^{b}, R^{c}, R^{d} and R^{e}, whose meaning is described hereinbefore.

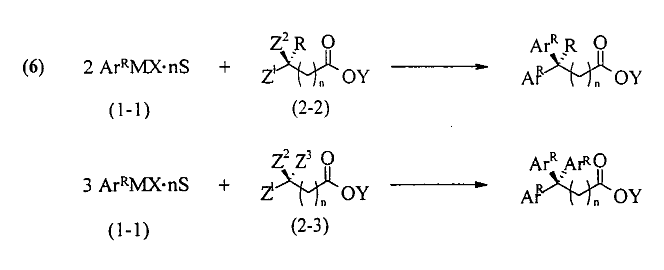

The process of this invention can be preferably performed as an one-pot process within the temperature range between -80°C and 200°C, preferably between -20°C and 150°C and most preferably between 20°C and 70°C.

The ratio between the halo-fluoroaryl and the haloacetate can be between 1:1 and 2:1 , preferably between 1.2:1 and 1.5 :1.

The ratio of the two metals, involved in the transmetalation is between 2:1 and 1:2, preferably 1:1.

The concentration of the Ni(II)-catalyst can be between 0.01mol% and 5mol%, preferably between 0.05mol% and 1mol%.

Preferred ligands for the stable Ni(II)-catalysts are those derived from α,β-diketones and bipyridyls, as they have an excellent selectivity towards the desired phenylacetic acid esters, formed with moderate to good yields with reduced or negligable formation of unwanted biaryls, whereas those Ni(II)-catalysts, containing phosphine ligands lead to lower selectivities and yields of phenylacetic acid esters and increased formation of undesired biaryls. The most preferred Ni-catalysts can be added as defined complexes in the preferred concentrations to the reaction mixtures, they can be also formed in-situ from Ni(II)-salts and the preferred ligands in the appropriate stoichiometric relation by adding these components to the reaction mixtures after the transmetalation step and before the addition of the haloacetate.

Preferred embodiments of the process according to the present invention are described in the attached claims 2 to 9.

### Example 1

A solution of 1-bromo-3,4,5-trifluorobenzene (0.132mol, 27.8g) in THF is added dropwise to a suspension of magnesium (0.132mol, 3.2g) in THF. The mixture is refluxed for 30min after the exothermic reaction has faded away. The Grignard solution is added dropwise to a stirred solution of anhydrous zinc chloride (0.132mol, 18g) in THF, which is cooled to 0°C. When the addition is completed, the solution is still stirred at room temperature for 30min. The reaction mixture is cooled to 0°C, ethyl bromoacetate (0.11mol, 18.4g) and the catalyst bis(acetylacetonato)-2,2'-bipyridinenickel(II) (0.4mmol, 219 mg, 0.5mol%) is added. The reaction is kept at 5°C overnight. The solution is quenched with ice/water and extracted with diethyl ether. The organic layer is washed with saturated sodium chloride solution. After drying over sodium sulfate, the solvent is evaporated. The crude product is purified by distillation, isolated yield: 19.1g, 80%, boiling point (10⁻²mbar) : 110°C.
GC (purified): 100% ethyl 3,4,5-trifluorophenylacetate
GPC: (RI) 100% ethyl 3,4,5-trifluorophenylacetate
MS: [m/z] 218 (M⁺, C₁₀H₉F₃O₂) ; 145 (base, M⁺-C₃H₅O₂) ; 125 (M⁺-C₃H₆FO₂); 29 (M⁺-C₈H₄F₃O₂)
IR: [cm⁻¹] 3459/2ν (C=O); 3081/ν(CH-Ar); 2987; 2942/ν(CH₂/CH₃) ; 1738/ν(C=O); 1622; 1532/ν(C=C); 1451/δ(CH-Alk); 1259/ν(CF-Ar); 1263/νₐₛ, (COO) ; 1046/νₐₛ, (OCHR) ; 876; 854/γₒₒₚ (isolated H-Ar)
¹H-NMR [ppm] 1.22 (CH₃-Et, t, 3H); 3.7 (CH₂-COO, s, 2H); 4.14 (CH₂-Et, q, 2H); 7.17 (CH-Ar, s, 2H) *(acetone-d6, 25°C, 300MHz)* ¹³C-NMR [ppm] 14.4 (CH₃-Et); 40.2 (CH₂-COO); 61.4 (CH₂-Et); 112.3; 112.6 (side product); 114.6; 114.7; 114.8; 114.9 (C2-Ar, ²J (C, F) =15Hz) ; 133 (Cl-Ar); 137.7; 141 (C3/C5-Ar, ¹J(C, F) =248Hz) ; 149.8; 153.1 (C4-Ar, ¹J(C,F)=247Hz); 170.8 (C1-Ar) *(acetone-d6, 25°C, 75. 43MHz)*
¹⁹F-NMR [ppm] -146 (F-C3/C5-Ar); -174.2 (F-C4-Ar) *(acetone-d6, 25 °C, 282.26MHz)*

### saponification:

An emulsion of 0.06mol, 13.1g ethyl 3,4,5-trifluorophenylacetate and 100ml 1.35M aqueous NaOH solution (5.4g, 0.135mol, 2eq) is refluxed for 3h. 4 equivalents HCl (diluted aqueous solution) are added after cooling to room temperature. The white precipitate of 3,4,5-trifluorophenylacetic acid (8.7g) is filtered off and recrystallized from hexane (7.7g) (melting point: 100°C).
GC: 100% 3,4,5-trifluorophenylacetic acid
GPC: RI, UV 100% 3,4,5-trifluorophenylacetic acid
MS: (m/z) 190 (M⁺, C₈H₅F₃O₂) ; 145 (base, M⁺-CHO₂) ; 125 (M⁺-CH₂FO₂) IR (nujol): [cm⁻¹] ~3000/ν(OH); 3084; 3061/ν(CH-Ar); 1717/ν(C=O); 1624; 1531/ν(C=C); 1326/δ(CH₂); 1249/ν(C-F-Ar); 1232/νₐₛ (COO); 920/δₒₒₚ, (OH); 857/γ (isolated H-Ar); 722/δ (CH₂) ¹H-NMR [ppm] 2.1 (CH₂-COO, s, 2H); 7.19 (CH-Ar, m, 2H) *(acetone-d6, 25 °C, 300MHz)*
¹³C-NMR [ppm] 39.9 (CH₂-COO); 114.8 (C2-Ar, ²J(C,F) = 15Hz); 133 (C1-Ar, ³J(C,F)=5Hz); 137.6; 140.9 (C3/C5-Ar, ¹J(C,F)=248Hz); 149.8; 153.1 (C4-Ar, ¹J(C,F)=249Hz); 171.8 (C1-Ar) *(acetone-d6, 25 °C, 75.43MHz)*
¹⁹F-NMR [ppm] -146 (F-C3/C5-Ar); -174,5 (F-C4-Ar) *(acetone-d6, 25 °C, 282. 26MHz)*

### Example 2

A solution of n-butyl lithium (1.3mmol) in hexane is added dropwise to a stirred solution of 1,2-difluorobenzene in THF which is cooled to -78°C. After stirring 1h at -78°C a solution of anhydrous zinc chloride (1.3mmol) in THF is added dropwise and the mixture is warmed up to room temperature. The solution is stirred at room temperature for 1h and cooled to 0°C. Ethyl bromoacetate (1.3mmol) and the catalyst bis(acetylacetonato)-2,2'-bipyridinenickel(II) (0.4mmol/0.5mol%) are added, afterwards the reaction is kept at room temperature overnight. The solution is quenched with ice/water and extracted with diethyl ether. The organic layer is washed with saturated sodium chloride solution. After drying over sodium sulfate the solvent is evaporated. The crude product is purified by distillation. yield 77%

### Example 3

By the process of example 2, 1,2-difluorobenzene and ethyl bromoacetate yield ethyl 2-(2,3-difluorophenyl)acetate. Differing from example 2, the molar ratio of the aryl compound and the ester is 1.5:1, the reaction time is 1h. yield 74%

### Example 4

By the process of example 2, 1,2-difluorobenzene and ethyl bromoacetate yield ethyl 2-(2,3-difluorophenyl)acetate, using bis(acetylacetonato)-4,4'-dimethoxy-2,2'-bipyridinenickel(II). yield 60%

### Example 5

By the process of example 2, 1,2-difluorobenzene and ethyl bromoacetate yield ethyl 2-(2,3-difluorophenyl)acetate. Differing from example 2, the molar ratio of the aryl compound and the ester is 1.5:1 and the reaction temperature is 50°C. yield 65%

### Example 6

By the process of example 2, 1,2-difluorobenzene and ethyl bromoacetate yield ethyl 2-(2,3-difluorophenyl)acetate, using bis(acetylacetonato)-4,4'-dimethyl-2,2'-bipyridinenickel(II) as catalyst. yield 18%

### Example 7

By the process of example 4, 1,2-difluorobenzene and ethyl bromoacetate yield ethyl 2-(2,3-difluorophenyl)acetate, the reaction temperature is 5°C. yield 46%

### Example 8

By the process of example 2, 1,2-difluorobenzene and ethyl bromoacetate yield ethyl 2-(2,3-difluorophenyl)acetate, using bis(acetylacetonato)-bis(perfluorotriphenylphosphine)-nickel(II) as catalyst. Differing from example 2, the molar ratio of the aryl compound and the ester is 1.5:1. yield 10%

### Example 9

By the process of example 2, 1,2-difluorobenzene and ethyl bromoacetate yield ethyl 2-(2,3-difluorophenyl)acetate, using bis(acetylacetonato)-bis(triphenylphosphine)nickel(II) as catalyst. Differing from example 2, the molar ratio of the aryl compound and the ester is 1.5:1. yield 61%

### Example 10

By the process of example 2, 1,2-difluorobenzene and ethyl bromoacetate yield ethyl 2-(2,3-difluorophenyl)acetate, using bis(acetylacetonato)-bis[tris-(o-tolyl)phosphine]-nickel(II) as catalyst. Differing from example 2, the molar ratio of the aryl compound and the ester is 1.5:1. yield 10%

### Example 11

By the process of example 2, 1,2-difluorobenzene and ethyl bromoacetate yield ethyl 2-(2,3-difluorophenyl)acetate, using bis(acetylacetonato)-bis[tris-(p-methoxyphenyl)phosphine]-nickel(II) as catalyst. Differing from example 2, the molar ratio of the aryl compound and the ester is 1.5:1. 45%

### Example 12

By the process of example 2, 1,2-difluorobenzene and ethyl bromoacetate yield ethyl 2-(2,3-difluorophenyl)acetate, using bis(acetylacetonato)-bis[tris-(2,6-dimethoxyphenyl)phosphine]-nickel(II) as catalyst. Differing from example 2, the molar ratio of the aryl compound and the ester is 1.5:1. yield 8%

### Example 13

By the process of example 2, 1,2-difluorobenzene and ethyl bromoacetate yield ethyl 2-(2,3-difluorophenyl)acetate, using bis(acetylacetonato)-bis(diphenylphosphine)nickel(II) as catalyst. Differing from example 2, the molar ratio of the aryl compound and the ester is 1.5:1. yield 57%

### Example 14

By the process of example 2, 1,2-difluorobenzene and ethyl bromoacetate yield ethyl 2-(2,3-difluorophenyl)acetate, using bis(acetylacetonato)-bis(dicyclohexylphenylphosphine)-nickel(II) as catalyst. Differing from example 2, the molar ratio of the aryl compound and the ester is 1.5:1. yield 60%.

### Example 15

By the process of example 2, 1,2-difluorobenzene and ethyl bromoacetate yield ethyl 2-(2,3-difluorophenyl)acetate, using bis(acetylacetonato)-bis(tricyclohexylphosphine)nickel(II) as catalyst. Differing from example 2, the molar ratio of the aryl compound and the ester is 1.5:1. yield 20%

### Example 16

By the process of example 2, 1,2-difluorobenzene and ethyl bromoacetate yield ethyl 2-(2,3-difluorophenyl)acetate, using dichloro-bis[-1,2-(diphenylphosphino)ethane]nickel(II) as catalyst. Differing from example 2, the molar ratio of the aryl compound and the ester is 1.5:1. yield 20%

### Example 17

By the process of example 2, 1,2-difluorobenzene and ethyl bromoacetate yield ethyl 2-(2,3-difluorophenyl)acetate, using bis(acetylacetonato)-[1,1'-(diphenylphosphino)ferrocene]-nickel(II) as catalyst. Differing from example 2, the molar ratio of the aryl compound and the ester is 1.5:1. yield 40%

### Example 18

By the process of example 2, 1,2-difluorobenzene and ethyl bromoacetate yield ethyl 2-(2,3-difluorophenyl)acetate, using bis(acetylacetonato)-1,10-(phenanthroline)nickel(II) as catalyst. Differing from example 2, the molar ratio of the aryl compound and the ester is 1.5:1. yield 45%

### Example 19

By the process of example 2, 1,2-difluorobenzene and ethyl bromoacetate yield ethyl 2-(2,3-difluorophenyl)acetate, using bis(acetylacetonato)nickel(II) as catalyst. Differing from example 2, the molar ratio of the aryl compound and the ester is 1.5:1. yield 14%

### Example 20

By the process of example 1, 1-bromo-3,4,5-trifluorobenzene and ethyl bromoacetate yield ethyl 2-(3,4,5-trifluorophenyl)acetate. Differing from example 1, the reaction temperature is room temperature. yield 92%

### Example 21

By the process of example 1, 1-bromo-3,4,5-trifluorobenzene and ethyl bromoacetate yield ethyl 2-(3,4,5-trifluorophenyl)-acetate. Differing from example 1, the reaction time is 45min. yield 92%

### Example 22

By the process of example 1, 1-bromo-3,4,5-trifluorobenzene and ethyl bromoacetate yield ethyl 2-(3,4,5-trifluorophenyl)-acetate, using dichloro-[1,1'-(diphenylphosphino)ferrocene]palladium(II) as catalyst. yield 20%

### Example 23

By the process of example 1, 1-bromo-3,4,5-trifluorobenzene and ethyl bromoacetate yield ethyl 2-(3,4,5-trifluorophenyl)acetate, using dichloro-bis(triphenylphosphine)nickel(II) as catalyst. Differing from example 1, the reaction time is 1h. yield 10%

### Example 24

By the process of example 1, 1-bromo-3,4,5-trifluorobenzene and ethyl bromoacetate yield ethyl 2-(3,4,5-trifluorophenyl)acetate, using dichloro-bis[1,2-(diphenylphosphino)-ethane]nickel(II) Differing from example 1, the reaction temperature is room temperature. yield 29%

### Example 25

By the process of example 1, 1-bromo-3,4,5-trifluorobenzene and ethyl bromoacetate yield ethyl 2-(3,4,5-trifluorophenyl)acetate, using bis(acetylacetonato)-[1,1'-(diphenylphosphino)ferrocene]-nickel(II) as catalyst. Differing from example 1, the reaction time is 1h. yield 44%

### Example 26

By the process of example 1, 1-bromo-3,4,5-trifluorobenzene and ethyl bromoacetate yield ethyl 2-(3,4,5-trifluorophenyl)acetate, using bis(acetylacetonato)-2,2'-bisoxazolinenickel(II) as catalyst. Differing from example 1, the reaction temperature is room temperature. yield 50%

### Example 27

By the process of example 1, 1-bromo-3,4,5-trifluorobenzene and ethyl bromoacetate yield ethyl 2-(3,4,5-trifluorophenyl)acetate, using bis(acetylacetonato)-2,2'-bisoxazolinenickel(II) as catalyst. Differing from example 1, the reaction time is 1h. yield 51%

### Example 28

By the process of example 1, 1-bromo-3,4,5-trifluorobenzene and ethyl bromoacetate yield ethyl 2-(3,4,5-trifluorophenyl)acetate, using bis(acetylacetonato)-[1,1'-(diphenylphosphino)ferrocene]-nickel(II) as catalyst. Differing from example 1, the reaction temperature is room temperature. yield 45%

### Example 29

By the process of example 1, 1-bromo-3,4,5-trifluorobenzene and ethyl bromoacetate yield ethyl 2-(3,4,5-trifluorophenyl)acetate, using bis(acetylacetonato)-bis(triphenylphosphite)nickel(II) as catalyst. Differing from example 1, the reaction temperature is room temperature. yield 60%

### Example 30

By the process of example 24, 1-bromo-2,4-difluorobenzene and ethyl bromoacetate yield ethyl 2,4-difluorophenylacetate, using dichloro-1,2-bis(diphenylphosphino)ethanenickel(II) as catalyst. yield 28%

### Example 31

By the process of example 24, 1-bromo-2,4-difluorobenzene and ethyl bromoacetate yield ethyl 2,4-difluorophenylacetate, using bis(acetylacetonato)-1,10-phenanthrolinenickel(II) as catalyst. yield 57%

### Example 32

By the process of example 24, 1-bromo-2,4-difluorobenzene and ethyl bromoacetate yield ethyl 2,4-difluorophenylacetate, using dichloro-1,2-bis(diphenylphosphino)ethanepalladium(II) as catalyst. yield 27%

### Example 33

By the process of example 24, 1-bromo-2,4-difluorobenzene and ethyl bromoacetate yield ethyl 2,4-difluorophenylacetate, using bis(acetylacetonato)-2,2'-bipyridinenickel(II) as catalyst. yield 85%

### Example 34

By the process of example 24, 1-bromo-2,4-difluorobenzene and ethyl bromoacetate yield ethyl 2,4-difluorophenylacetate, using bis(acetylacetonato)-2,2'-bipyridinenickel(II) as catalyst. Differing from example 24, the reaction temperature is 50°C. yield 52%

### Example 35

By the process of example 24, 1-bromo-2,4-difluorobenzene and ethyl bromoacetate yield ethyl 2,4-difluorophenylacetate, using bis(acetylacetonato)-4,4'-dimethyl-2,2'-bipyridinenickel(II) as catalyst. yield 57%

### Example 36

By the process of example 24, 1-bromo-2,4-difluorobenzene and ethyl bromoacetate yield ethyl 2,4-difluorophenylacetate, using bis(acetylacetonato)-4,4'-dimethoxy-2,2'-bipyridinenickel(II) as catalyst. yield 60%

### Example 37

By the process of example 24, 1-bromo-3,5-difluorobenzene and ethyl bromoacetate yield ethyl 2-(3,5-difluorophenyl)acetate, using bis(acetylacetonato)-2,2'-bipyridinenickel(II) as catalyst. yield 94%

### Example 38

By the process of example 37, 1-bromo-3,4-difluorobenzene and ethyl bromoacetate yield ethyl 2-(3,4-difluorophenyl)acetate. yield 87%

### Example 39

By the process of example 37, 1-bromo-3-fluorobenzene and ethyl bromoacetate yield ethyl 2-(3-fluorophenyl)acetate. yield 72%

### Example 40

By the process of example 37, 1-bromo-4-fluorobenzene and ethyl bromoacetate yield ethyl 2-(4-fluorophenyl)acetate. yield 74%

### Example 41

By the process of example 37, 1-bromo-3,4-difluorobenzene and ethyl 3-bromopropionate yield ethyl 3-(3,4-difluorophenyl)propionate, using bis(acetylacetonato)-2,2'-bisoxazolinenickel(II) as catalyst. yield 11%

### Example 42

By the process of example 41, 1-bromo-3,4-difluorobenzene and ethyl 3-iodopropionate yield ethyl 3-(3,4-difluorophenyl)propionate. yield 55%

### Example 43

By the process of example 35, 1-bromo-3,4-difluorobenzene and ethyl 3-iodopropionate yield ethyl 3-(3,4-difluorophenyl)propionate, using bis(acetylacetonato)-2,2'-bipyridinenickel(II) as catalyst. yield 45%

### Example 44

By the process of example 35, 1-bromo-3,4-difluorobenzene and ethyl 3-iodopropionate yield ethyl 3-(3,4-difluorophenyl)propionate, using bis(acetylacetonato)-2,2'-bisoxazolinenickel(II) as catalyst. yield 55%

### Example 45

By the process of example 35, 1-bromo-3,4-difluorobenzene and ethyl 3-iodopropionate yield ethyl 3-(3,4-difluorophenyl)propionate, using bis(acetylacetonato)bis(tricyclohexyl)nickel(II) as catalyst. yield 10%

### Example 46

By the process of example 35, 1-bromo-3,4-difluorobenzene and ethyl 3-iodopropionate yield ethyl 3-(3,4-difluorophenyl)propionate, using bis(acetylacetonato)bis(tri-n-octyl)nickel(II) as catalyst. yield 28%

## Claims

1. A process for the preparation of fluorophenylalkylencarboxylic acid derivatives (4-1) to (4-3): 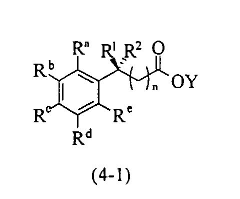 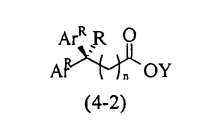 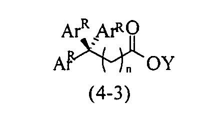 wherein R^{a} to R^{e} are H, F, alkyl, alkoxy, fluoroalkyl, cycloalkyl, fluorocycloalkyl, aryl, fluoroaryl, alkenyl, alkynyl,
R, R¹ and R² are hydrogen atoms or alkyl, cycloalkyl, aryl, alkenyl and alkynyl, which can be substituted by alkyl and alkoxy groups,
n is from 0 to 10,
Y is alkyl, aryl, Li, Na, K or a group II metal (except Ra),
Ar^{R} is an aryl group substituted by H, F, alkyl, fluoroalkyl, cycloalkyl, fluorocycloalkyl, aryl, fluoroaryl, alkenyl, alkynyl;
wherein fluoroarylmetal halides (Ar^{R}M^{a,b}X)_{y}·nS (1-1) resp. (Ar^{R}M^{a,b}M^{c})_{y} · nS (1-2) : 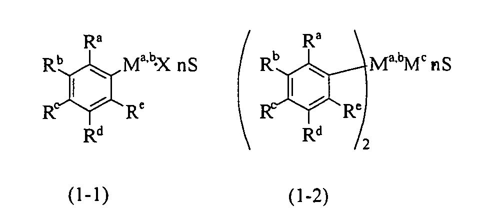 wherein R^{a}, R^{b}, R^{c}, R^{d} and R^{e} are as defined above, wherein in (1-1) M^{a} and M^{b} are Li, Mg, Zn, Cu(II) and in (1-2) M^{a} and M^{b} are Li, Mg or Zn, X is a Cl, Br or I, S is an optionally substituted dialkyl or alicyclic ether
are reacted with carboxylic acid derivatives RₚZ₍₃₋ₚ₎(CH₂)ₙCOOY (2-1)~(2-3) 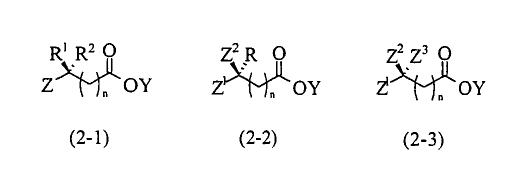 wherein Z, Z¹, Z² and Z³ are Cl, Br or I, n and Y are as defined above, p is 0 to 3,
in the presence of at least a transition metal catalyst LₙTMX¹X² selected from the group (3-1)~(3-6) 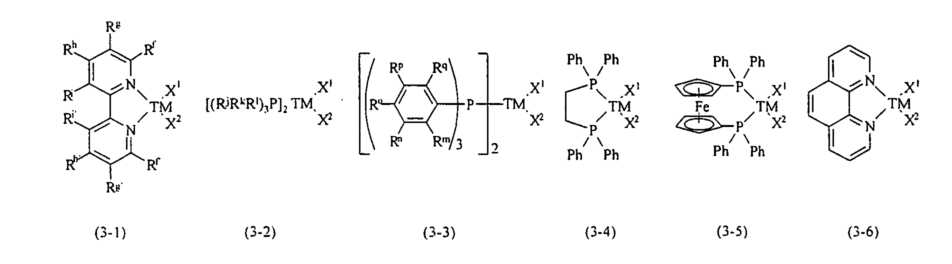
LₙTMXₘ (3-7)
wherein TM is divalent or zerovalent nickel, palladium or platinum,
R^{f}, R^{g}, R^{h}, Rⁱ, R^{f'}, R^{g'}, R^{h'} and R^{i'} are hydrogen, alkyl, alkoxy, perfluoroalkyl, perfluoroalkoxy, alkenyl, alkynyl or halogen (except At),
R^{j}, R^{k} and R^{l} are alkyl, alkoxy, perfluoroalkyl, perfluoroalkoxy, cycloalkyl, cycloalkoxy, perfluorocycloalkyl and perfluorocycloalkoxy.
R^{m}, Rⁿ, R^{o}, R^{p} and R^{q} are hydrogen, alkyl, alkoxy, perfluoroalkyl, perfluoroalkoxy, alkenyl, alkynyl or halogen (except At),
and wherein, when TM is divalent, X¹ and X² are inorganic ionic ligands,
and when TM is zerovalent, X¹ and X² are inorganic or organic donorligands,
and wherein in (3-7) m and n are integers from 1 to 6, the ligand L is alkyl phosphine, aryl phosphine, alkyl aryl phosphine, bipyridyl or phenanthroline, substituted by hydrogen, alkyl, alkoxy, perfluoroalkyl, perfluoroalkoxy, cycloalkyl, cycloalkoxy, perfluorocycloalkyl and perfluorocycloalkoxy, alkenyl, alkynyl, chlorine, bromine or iodine;
the ligands can be bridged by substituted or unsubstituted alkyl groups, aryl groups and ferrocene;
in case TM is a divalent metal, X is an inorganic ionic ligand like chloride, bromide, iodide, hydride, sulfate, sulfite, nitrate, nitrite, amide or amide substituted by alkyl, cycloalkyl, aryl or organic carboxylate;
X is alkyl, cycloalkyl, aryl or part of an ionic chelate ligand;
in case TM is a zerovalent metal, X can be an inorganic or organic donor ligand.

2. The process according to claim 1, wherein (Ar^{R}M^{a,b}X)_{y}·nS (1-1) resp. (Ar^{R}M^{a,b}M^{c})_{y}·nS
n and y are whole numbers from 1 to 8;
S is a substituted or unsubstituted alicyclic ether consisting of a five membered ring that is composed of carbon atoms except one, which is oxygen or
consisting of a six membered carbon ring that bears a nitrogen instead of the carbon atom in position 4, which can be substituted by H, alkyl, fluoroalkyl, cycloalkyl, fluorocycloalkyl, aryl, fluoroaryl, alkenyl, alkynyl;
in (1-1) and (1-2) M^{a} is Li, Mg, with the proviso that the metal is not X-substituted if M is Li;
in (1-1) M^{b} is Mg (except M^{a} is Mg), Zn, Cu(II);
in (1-2) M^{b} is Mg (except M^{a} is Mg), Zn;
in (1-2) M^{c} is Cu(I), and X is chlorine, bromine or iodine.

3. The process according to claim 1, wherein in the carboxylic acid derivative RpZ₍₃₋ₚ₎ (CH₂)ₙCOOY (2-1)~(2-3)
Z is chlorine, bromine and/or iodine;
Y is selected from the group consisting of substituted or unsubstituted alkyl, aryl, lithium, sodium, potassium, beryllium, magnesium, calcium, strontium or barium;
m and n are whole numbers, m is from 0 to 3, n is from 0 to 10, preferably 0 or 1.

4. The process according to claim 1, wherein the compound S serves as reaction medium.

5. The process according to claims 1 to 4 wherein a saturated cyclic, aromatic or non-cyclic hydrocarbon or a mixture thereof is used as reaction medium.

6. The process according to claims 1 to 4, wherein THF is used as the reaction medium.

7. The process according to claim 1, wherein all transmetalation steps and the catalytical coupling reaction in the preparation of the fluoroarylmetal halides (Ar^{R}M^{a,b}X)_{y}·nS (1-1) and (Ar^{R}M^{a,b}M^{c})_{y}·nS (1-2) are carried out as a one-pot process.

8. The process according to claim 1, which is carried out in the range of temperature from -80 °C to 200°C, preferably from 20°C to 70°C.

9. The process according to claim 1, wherein the concentration of the transition metal catalyst is from 0.01mol% to 5mol%, preferably from 0.05mol% to 1mol%.
